Erfinder: **Gado, Klara, Dr.**
**Torbagy u. 13**
**Budapest XI(HU)**
Erfinder: **Hegedüs, Maria**
**Kérö u. 6**
**H-1112 Budapest(HU)**

(74) Vertreter: **Beszédes, Stephan G., Dr.**
**Münchener Strasse 80a Postfach 1168**
**D-8060 Dachau(DE)**

(54) **Benzyldiaminopyrimidin-Derivate und deren Verwendung als Arzneimittel.**

(57) Gegenstand der Erfindung ist die Verwendung von 5-(substituierten Benzyl) 2,4-Di-(amino)-pyrimidinderivaten der allgemeinen Formel

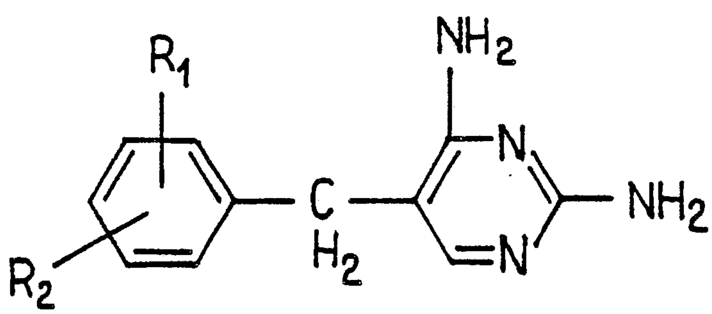

I ,

worin
$R_1$ und $R_2$ , die gleich oder verschieden sein können, für Wasserstoffatome, Hydroxygruppen, Alkoxyreste mit 1 bis 6 Kohlenstoffatom(en), Alkoxyalkoxyreste mit 1 bis 6 Kohlenstoffatom(en) in jedem Alkoxyteil, Alkenyloxyreste mit 2 bis 6 Kohlenstoffatomen beziehungsweise Phenylalkoxyreste mit 1 bis 3 Kohlenstoffatom(en) im Alkoxyteil stehen oder
$R_1$ und $R_2$ zusammen einen Alkylendioxyrest mit 1 oder 2 Kohlenstoffatom(en) darstellen,
mit der weiteren Maßgabe, daß
mindestens 1 von $R_1$ und $R_2$ von Wasserstoff verschieden ist,
sowie ihre Säureadditionssalze zur Herstellung von antipyretisch, entzündungshemmend einschließlich antirheumatisch, anti-anginös und/oder antioxydierend wirkenden Arzneimitteln.
Gegenstand der Erfindung sind auch die neuen Verbindungen unter diesen.

(19) Europäisches Patentamt
European Patent Office
Office européen des brevets

(11) Veröffentlichungsnummer: **0 301 428 A2**

# (12) EUROPÄISCHE PATENTANMELDUNG

(21) Anmeldenummer: **88111830.1**

(51) Int. Cl.⁴: **A61K 31/505**

(22) Anmeldetag: **22.07.88**

Die Bezeichnung der Erfindung wurde geändert (Richtlinien für die Prüfung im EPA, A-III, 7.3).

Patentansprüche für folgende Vertragsstaaten: ES + GR.

(30) Priorität: **22.07.87 HU 337587**

(43) Veröffentlichungstag der Anmeldung: **01.02.89 Patentblatt 89/05**

(84) Benannte Vertragsstaaten: **AT BE CH DE ES FR GB GR IT LI NL SE**

(71) Anmelder: **EGIS GYOGYSZERGYAR**
**Kereszturi ut 30-38**
**Budapest X(HU)**

(72) Erfinder: **Petöcz, Lujza, Dr.**
**Rakoczi tér 2**
**Budapest VIII(HU)**
Erfinder: **Simonyi, Istvan, Dr.**
**Néphadesereg u. 18/20**
**Budapest V(HU)**
Erfinder: **Beck, Ivan, Dr.**
**Zalai ut 1/d**
**Budapest XII(HU)**
Erfinder: **Gigler, Gabor**
**Lenin krt. 95**
**Budapest VI(HU)**
Erfinder: **Fekete, Marton, Dr.**
**Fö u. 49**
**Budapest II(HU)**
Erfinder: **Szirt geb. Kiszelly, Enikö**
**Téglavetö u. 24**
**Budapest X(HU)**
Erfinder: **Mandi, Attila, Dr.**
**Endrödi S. u. 24**
**Budapest II(HU)**
Erfinder: **Görgényi, Frigyes, Dr.**
**Szakasits A. u. 60/a**
**H-1115 Budapest(HU)**
Erfinder: **Dietz, Andras**
**Pitvar u. 11**
**Budapest XIV(HU)**
Erfinder: **Jakfalvi, Elemér**
**Pozsonyi ut 6**
**Budapest XVI(HU)**
Erfinder: **Zukovics geb. Sümeg, Katalin**
**Baranyai ut 9**
**Budapest XI(HU)**

EP 0 301 428 A2

# Verwendung von 5-(substituierten Benzyl) 2,4-Di-(amino)-pyrimidinderivaten und 5-(substituierte Benzyl) 2,4-Di-(amino)-pyrimidinderivate

Die Erfindung betrifft die Verwendung von 5-(substituierten Benzyl) 2,4-Di-(amino)-pyrimidinderivaten und 5-(substituierte Benzyl) 2,4-Di-(amino)-pyrimidinderivate, insbesondere zur Verwendung als Arzneimittel.

Es sind bereits 5-(substituierte Benzyl) 2,4-Di-(amino)-pyrimidinderivate bekannt (DE-OS 22 12 642, 23 63 533 und 21 65 363; AU-PS 308 727; HU-PS 149 799). Diese weisen an der Benzylgruppe gegebenenfalls als Substituenten 1 oder mehr Alkoxyrest(e) und/oder Benzyloxyrest(e) oder einen 2 benachbarte Positionen des Benzolringes derselben überbrückenden Methylendioxyrest auf. Als Wirkungen dieser Verbindungen sind antibakterielle und coccidiostatische Wirkungen, eine Antimalariawirkung und eine die antibakterielle Wirkung der Sulfonamide potenzierende Wirkung angegeben.

Der Erfindung liegt die Aufgabe zugrunde, die Verwendung von 5-(substituierten Benzyl) 2,4-Di-(amino)-pyrimidinderivaten für andere Indikationen mit überlegenem Ergebnis und neue 5-(substituierte) 2,4-Di-(amino)pyrimidinderivate, welche auch für diese Indikationen eingesetzt werden können, zu schaffen.

Das Obige wurde überraschenderweise durch die Erfindung erreicht.

Gegenstand der Erfindung ist die Verwendung von 5-(substituierten Benzyl) 2,4-Di-(amino)-pyrimidinderivaten der allgemeinen Formel

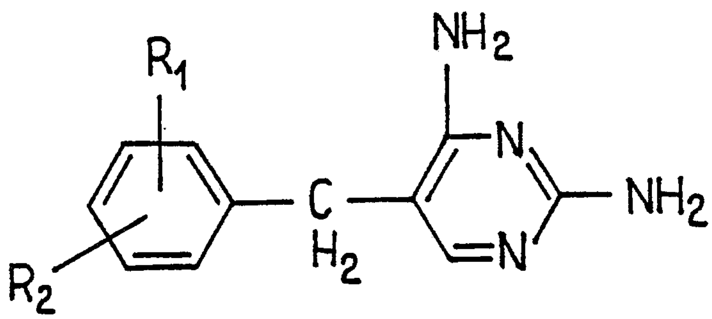

I ,

worin

$R_1$ und $R_2$ , die gleich oder verschieden sein können, für Wasserstoffatome, Hydroxygruppen, Alkoxyreste mit 1 bis 6 Kohlenstoffatom(en), Alkoxyalkoxyreste mit 1 bis 6 Kohlenstoffatom(en) in jedem Alkoxyteil, Alkenyloxyreste mit 2 bis 6 Kohlenstoffatomen beziehungsweise Phenylalkoxyreste mit 1 bis 3 Kohlenstoffatom(en) im Alkoxyteil stehen oder

$R_1$ und $R_2$ zusammen einen Alkylendioxyrest mit 1 oder 2 Kohlenstoffatom(en) darstellen,

mit der weiteren Maßgabe, daß

mindestens 1 von $R_1$ und $R_2$ von Wasserstoff verschieden ist, sowie ihre Säureadditionssalze zur Herstellung von antipyretisch, entzündungshemmend einschließlich antirheumatisch, anti-anginös und/oder antioxydierend wirkenden Arzneimitteln.

Ein Teil der vorstehend angegebenen 5-(substituierten Benzyl) 2,4-Di-(amino)-pyrimidinderivate ist neu. Gegenstand der Erfindung sind daher auch 5-(substituierte) 2,4-Di-(amino)-pyrimidinderivate der allgemeinen Formel

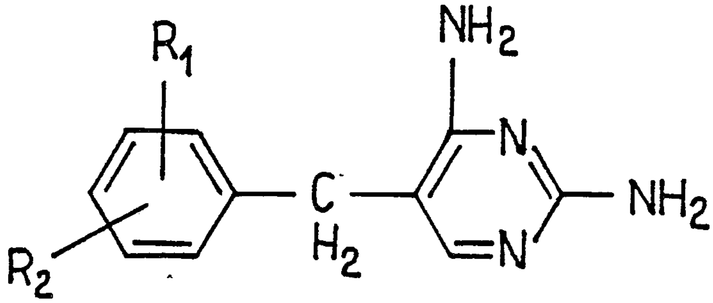

I ,

worin
$R_1$ und $R_2$ , die gleich oder verschieden sein können, für Wasserstoffatome, Hydroxygruppen, Alkoxyreste mit 1 bis 6 Kohlenstoffatom(en), Alkoxyalkoxyreste mit 1 bis 6 Kohlenstoffatom(en) in jedem Alkoxyteil, Alkenyloxyreste mit 2 bis 6 Kohlenstoffatomen beziehungsweise Phenylalkoxyreste mit 1 bis 3 Kohlenstoffatom(en) im Alkoxyteil stehen oder
$R_1$ und $R_2$ zusammen einen Äthylendioxyrest darstellen,
mit der weiteren Maßgabe, daß
im Falle daß
das eine von $R_1$ und $R_2$ für Wasserstoff oder einen Alkoxy-oder Benzyloxyrest steht,
das andere von $R_1$ und $R_2$ von Wasserstoff oder einem Alkoxy- oder Benzyloxyrest verschieden ist,
sowie ihre Säureadditionssalze, insbesondere zur Verwendung als Arzneimittel.

Wie es überraschenderweise festgestellt wurde, ist die analgetische und entzündungshemmende Wirksamkeit (therapeutischer Index) der 2,4-Di-(amino)-pyrimidinderivate der allgemeinen Formel I beziehungsweise ihrer Säureadditionssalze um bis zu 2 Größenordnungen höher als die entsprechenden Werte des auf diesem Gebiet anerkannten Handelsproduktes 1-[4′-(Chlor)-benzoyl]-5-[methoxy]-2-[methyl]-indol-3-ylessigsäure [Indometacin]. Diese Wirkung ist mit einer der Wirksamkeit der Acetylsalicylsäure entsprechenden antipyretischen Wirkung verbunden und gleichzeitig durch eine Antioxydationswirkung, welche der Wirksamkeit der Vergleichsprodukte gleich oder um bis zu 2 Größenordnungen stärker ist, ergänzt. Ein besonderer Vorteil der 2,4-Di-(amino)-pyrimidinderivate der allgemeinen Formel liegt darin, daß diese Verbindungen im Gegensatz zu den bekannten Analgetica und Antiphlogistica auf die Magenschleimhaut keine ungünstigen schädigenden Nebenwirkungen ausüben. Solche Nebenwirkungen treten nicht einmal in toxischen Dosen auf. Die Mehrzahl der 2,4-Di-(amino)-pyrimidinderivate der allgemeinen Formel I beziehungsweise ihrer Säureadditionssalze zeigt an Ratten eine die Wirk samkeit des N-[3,3-Di-(phenyl)-propyl]-α-[methyl]-phenyläthylamines [Prenylamines] übertreffende anti-anginöse Wirkung.

Vorzugsweise sind die 2,4-Di-(amino)-pyrimidinderivate solche, bei welchen der beziehungsweise die Alkoxyrest(e), für welche[n] $R_1$ und/oder $R_2$ stehen kann beziehungsweise können, [ein] solche[r] mit 1 bis 4, insbesondere 1 oder 2, Kohlenstoffatom(en) ist beziehungsweise sind.

Es ist auch bevorzugt, daß die 2,4-Di-(amino)-pyrimidinderivate solche, bei welchen der beziehungsweise die Alkoxyalkoxyrest(e), für welche[n] $R_1$ und/oder $R_2$ stehen kann beziehungsweise können, [ein] solche[r] mit 1 bis 4, insbesondere 1 und/oder 2, Kohlenstoffatom(en) in jedem Alkoxyteil, ganz besonders [ein] Methoxyäthoxyrest, vor allem 2-(Methoxy)-äthoxyrest, ist beziehungsweise sind, sind.

Ferner ist es bevorzugt, daß die 2,4-Di-(amino)-pyrimidinderivate solche, bei welchen der beziehungsweise die Alkenyloxyrest(e), für welche[n] $R_1$ und/oder $R_2$ stehen kann beziehungsweise können, [ein] solche[r] mit 2 bis 4, insbesondere 2 oder 3, Kohlenstoffatomen, ganz besonders [ein] Allylrest(e), ist beziehungsweise sind, sind.

Weiterhin ist es bevorzugt, daß die 2,4-Di-(amino)-pyrimidinderivate solche, bei welchen der beziehungsweise die Phenylalkoxyrest(e), für welche[n] $R_1$ und/oder $R_2$ stehen kann beziehungsweise können, [ein] solche[r] mit 1 oder 2 Kohlenstoffatom(en) im Alkoxyteil sind.

Zweckmäßig sind die Salze der 2,4-Di-(amino)-pyrimidinderivate der allgemeinen Formel I solche mit pharmazeutisch brauchbaren anorganischen oder organischen Säu ren, zum Beispiel Hydrochloride, Hydrobromide, Sulfate, Phosphate, Acetate, Maleate, Fumarate, Lactate, Ascorbinate, Citrate beziehungsweise Tartrate. Bevorzugt sind die Hydrochloride.

Besonders bevorzugt als 2,4-Di-(amino)-pyrimidinderivate der allgemeinen Formel sind
2,4-Di-[amino]-5-[4′-(methoxy)-benzyl]-pyrimidin,
2,4-Di-[amino]-5-[3′-(methoxy)-benzyl]-pyrimidin,
2,4-Di-[amino]-5-[3′-5′-di-(methoxy)-benzyl]-pyrimidin,
2,4-Di-[amino]-5-[2′,4′-di-(methoxy)-benzyl]-pyrimidin,
2,4-Di-[amino]-5-[2′,3′-di-(methoxy)-benzyl]-pyrimidin,
2,4-Di-[amino]-5-[3′,4′-(methylendioxy)-benzyl]-pyrimidin,
2,4-Di-[amino]-5-[3′-(methoxy)-4′-(äthoxy)-benzyl]-pyrimidin,
2,4-Di-[amino]-5-[3′-(methoxy)-4′-(allyloxy)-benzyl]-pyrimidin,
2,4-Di-[amino]-5-[3′-(methoxy)-4′-(n-butoxy)-benzyl]-pyrimidin,
2,4-Di-[amino]-5-[3′-(methoxy)-4′-(2″-{methoxy}-äthoxy)-benzyl]-pyrimidin,
2,4-Di-[amino]-5-[3′-(methoxy)-4′-(benzyloxy)-benzyl]-pyrimidin und/oder
2,4-Di-[amino]-5-[3′-(methoxy)-4′-(hydroxy)-benzyl]-pyrimidin
und/oder deren Säureadditionssalze.

Die pharmazeutische Wirksamkeit der 2,4-Di-(amino)-pyrimidinderivate der allgemeinen Formel I beziehungsweise ihrer Säureadditionssalze wurde auf Grund der folgenden Prüfversuche nachgewiesen.

1.) Akute Toxizität an Mäusen

Prüfverfahrensweise

Die Versuche wurden an männlichen und weiblichen CFLP-Mäusen (Körpergewicht 18 bis 22 g) durchgeführt. Jede Gruppe bestand aus je 10 Tieren. Die zu untersuchende Verbindung wurde in Lösung in 0,2 Gew.-% Tween®(80) enthaltendem Wasser peroral verabreicht. Die angewandte Dosis betrug 20 bis 30 ml/kg. Die Beobachtungsperiode dauerte 14 Tage nach der Verabreichung. Die statistische Auswertung wurde nach der Verfahrensweise von Litchfield-Wilcoxon vorgenommen (J. T. Litchfield, F. Wilcoxon: J. Pharmacol. Exp. Ther. 96 [1949], 99 bis 113). Die erhaltenen Ergebnisse sind in der folgenden Tabelle I zusammengestellt.

Tabelle I

Akute Toxizität an Mäusen

| Untersuchte Verbindung: Bezeichnung | Untersuchte Verbindung: Beispiel Nr. | $LD_{50}$-Wert in mg/kg |
|---|---|---|
| 2,4-Di-[amino]-5-[4'-(methoxy)-benzyl]-pyrimidin | 1 | etwa 350 |
| 2,4-Di-[amino]-5-[3'-(methoxy)-benzyl]-pyrimidin | 2 | etwa 410 |
| 2,4-Di-[amino]-5-[3',5'-di-(methoxy)-benzyl]-pyrimidin | 3 | etwa 800 |
| 2,4-Di-[amino]-5-[2',4'-di-(methoxy)-benzyl]-pyrimidin | 4 | etwa 2 000 |
| 2,4-Di-[amino]-5-[2',3'-di-(methoxy)-benzyl]-pyrimidin | 5 | etwa 500 |

Fortsetzung der Tabelle I

| Untersuchte Verbindung: Bezeichnung | Untersuchte Verbindung: Beispiel Nr. | $LD_{50}$-Wert in mg/kg |
|---|---|---|
| 2,4-Di-[amino]-5-[3',4'-(methylen-dioxy)-benzyl]-pyrimidin | 6 | etwa 750 |
| 2,4-Di-[amino]-5-[3'-(methoxy)-4'-(äthoxy)-benzyl]-pyrimidin | 7 | etwa 350 |
| 2,4-Di-[amino]-5-[3'-(methoxy)-4'-(allyloxy)-benzyl]-pyrimidin-hydrochlorid | 8 | etwa 1 500 |
| 2,4-Di-[amino]-5-[3'-(methoxy)-4'-(n-butoxy)-benzyl]-pyrimidin | 9 | > 2 000 |
| 2,4-Di-[amino]-5-[3'-(methoxy)-4'-2''-{methoxy}-äthoxy)-benzyl]-pyrimidin | 10 | < 500 |
| 2,4-Di-[amino]-5-[3'-(methoxy)-4'-(ben-zyloxy)-benzyl]-pyrimidin | 11 | > 2 000 |

Fortsetzung der Tabelle I

| Untersuchte Verbindung | | $LD_{50}$-Wert in mg/kg |
|---|---|---|
| Bezeichnung | Beispiel Nr. | |
| 2,4-Di-[amino]-5-[3'-(methoxy)-4'-(hydroxy)-benzyl]-pyrimidin-hydrobromid | 12 | > 2 000 |
| 1-[4'-(Chlor)-benzoyl]-5-[methoxy]-2-[methyl]-indol-3-ylessigsäure [Indometacin] | Vergleichssubstanz A | 22,5 |
| Acetylsalicylsäure | Vergleichssubstanz B | 1 350 |

2.) Analgetische Wirkung an Ratten "Schmerzkrümmungs-Versuch" "Writhing-Test"

Prüfverfahrensweise

Ratten (Körpergewicht 130 bis 170 g) wurde Essigsäure in einer Konzentration von 0,75 Vol.-% intraperitoneal in einer Dosis von 8 ml/kg verabreicht. 5 Minuten nach der Behandlung wurde die Gesamtzahl der typischen "Schmerzkrümmungs-Reaktionen" ("Writhing-Reaktionen") 10 Minuten lang bestimmt und in % der der Blindversuchsbeziehungsweise Kontrollgruppe ausgedrückt. Die Tiere wurden 1 Stunde von der Injizierung der Essigsäurelösung mit der zu untersuchenden Verbindung beziehungsweise der Trägersubstanz (Blindversuchs- beziehungsweise Kontrollgruppe) oral behandelt. Die Ergebnisse sind in der folgenden Tabelle II zusammengestellt.

**Tabelle II**

Analgetische Wirksamkeit an Ratten

| Untersuchte Verbindung: Bezeichnung | Untersuchte Verbindung: Beispiel Nr. | Therapeutischer Index |
|---|---|---|
| 2,4-Di-[amino]-5-[4'-(methoxy)-benzyl]-pyrimidin | 1 | etwa 8,1 |
| 2,4-Di-[amino]-5-[3'-(methoxy)-benzyl]-pyrimidin | 2 | etwa 5,9 |
| 2,4-Di-[amino]-5-[3',5'-di-(methoxy)-benzyl]-pyrimidin | 3 | etwa 8,0 |
| 2,4-Di-[amino]-5-[2',4'-di-(methoxy)-benzyl]-pyrimidin | 4 | etwa 33,3 |
| 2,4-Di-[amino]-5-[2',3'-di-(methoxy)-benzyl]-pyrimidin | 5 | etwa 5,0 |

Fortsetzung der Tabelle II

| Untersuchte Verbindung: Bezeichnung | Untersuchte Verbindung: Beispiel Nr. | Therapeutischer Index |
|---|---|---|
| 2,4-Di-[amino]-5-[3',4'-(methylen-dioxy)-benzyl]-pyrimidin | 6 | etwa 57,7 |
| 2,4-Do-[amino]-5-[3'-(methoxy)-4'-(äthoxy)-benzyl]-pyrimidin | 7 | etwa 7,6 |
| 2,4-Di-[amino]-5-[3'-(methoxy)-4'-allyloxy-benzyl]-pyrimidin-hydrochlorid | 8 | etwa 42,9 |
| 2,4-Di-[amino]-5-[3'-(methoxy)-4'-(n-butoxy)-benzyl]-pyrimidin | 9 | > 125,0 |
| 2,4-Di-[amino]-5-[3'-(methoxy)-4'-2''-{methoxy}-äthoxy)-benzyl]-pyrimidin | 10 | < 14,3 |
| 2,4-Di-[amino]-5-[3'-(methoxy)-4'-(ben-zyloxy)-benzyl]-pyrimidin | 11 | > 37,0 |

**Fortsetzung der Tabelle II**

| Untersuchte Verbindung: Bezeichnung | Untersuchte Verbindung: Beispiel Nr. | Therapeutischer Index |
|---|---|---|
| 2,4-Di-[amino]-5-[3'-(methoxy)-4'-(hydroxy)-benzyl]-pyrimidin-hydrobromid | 12 | > 40,0 |
| 1-[4'-(Chlor)-benzoyl]-5-[methoxy]-2-[methyl]-indol-3-ylessigsäure [Indometacin] | Vergleichssubstanz A | 4,8 |

Aus der obigen Tabelle II geht hervor, daß die 2,4-Di-(amino)-pyrimidinderivate eine wesentlich bessere analgetische Wirkung bei wesentlich höheren therapeutischen Indices um bis zu 2 Größenordnungen haben als die Vergleichssubstanz.

3.) Antipyretische Wirkung an Ratten

Prüfverfahrensweise

Die Versuche wurden an aus je 10 männlichen und weiblichen Wistar-Ratten (Körpergewicht: 160 bis 200 g) bestehenden Gruppen durchgeführt. Das Fieber wurde durch subkutane Injektion einer 20 Gew.-%-igen Hefesuspension in einer 0,9 gew.-%-igen Kochsalzlösung (Volumen: 2 ml//Tier) an verschiedenen Stellen des Rückens hervorgerufen. Nach 18 Stunden wurde die zu untersuchende Verbindung oder der Träger (Blind- beziehungsweise Kontrollversuch) oral in einer Dosis von 10 mg/kg verabreicht. Während dieser Periode wurden die Tiere nüchtern gehalten, erhielten jedoch Trinkwasser nach Belieben. Die rektale Temperatur der Tiere wurde 2 Tage vor der Verabreichung der zu untersuchenden Verbindung und nach der Behandlung jede Stunde mit einem Thermometer gemessen. Tiere mit einer Körpertemperaturerhöhung von weniger als 0,8° C wurden ausgeschlossen und nicht berücksichtigt. Die Ergebnisse wurden nach der statistischen Verfahrensweise von Duncan ausgewertet. Die Ergebnisse sind in der folgenden Tabelle III zusammengestellt.

Tabelle III

| Antipyretische Wirkung an Ratten | | | |
|---|---|---|---|
| Untersuchte Verbindung | | Dosis in mg/kg | Höchste Körpertemperaturerniedrigung in °C |
| Bezeichnung | Beispiel Nr. | | |
| 2,4-Di-[amino]-5-[4′-(methoxy)-benzyl]-pyrimidin | 1 | 200 | 0,9 |
| 2,4-Di-[amino]-5-[3′,4′-(methylendioxy)-benzyl]-pyrimidin | 6 | 100 | 1,0 |
| 2,4-Di-[amino]-5-[3′-(methoxy)-4′-(äthoxy)-benzyl]-pyrimidin | 7 | 100 | 0,7 |
| 2,4-Di-[amino]-5-[3′-(methoxy)-4′-(allyloxy)-benzyl]-pyrimidin-hydrochlorid | 8 | 100 | 0,8 |

Fortsetzung der Tabelle III

| Untersuchte Verbindung | | Dosis in mg/kg | Höchste Körpertemperaturerniedrigung °C |
|---|---|---|---|
| Bezeichnung | Beispiel Nr. | | |
| Acetylsalicylsäure | Vergleichssubstanz B | 200 | 1,0 |

Aus der obigen Tabelle III geht hervor, daß bei den 2,4-Di-(amino)-pyrimidinderivaten , die sich zusätzlich zur überlegenen analgetischen Wirkung zeigende antipyretische Wirkung der ersteren der der Acetylsalicylsäure gleich ist.

4.) Chemilumineszierende Wirkung

Prüfverfahrensweise

Die 2,4-Di-(amino)-pyrimidinderivate wurden in Dimethylsulfoxyd zu einer Konzentration von $10^{-1}$ Mol/1 gelöst; Verdünnungen wurden im Inkubationsmedium hergestellt.

Es wurde hepariniertes Blut (10 IE/ml) gesunden Freiwilligen entnommen. Das Blut wurde mit dem gleichen Volumen Dextran verdünnt; die Leukocyten wurden durch Sedimentieren abgetrennt. Die Granulocyten wurden durch Uromio-Gradienten getrennt. Die mononuklearen Zellen wurden durch Zentrifugieren in Ficoll-Uromio-Gradienten (Dichte 1,078 g/ml; 30 Min., Drehzahl 2 000 $min^{-1}$) getrennt. Die Zellen wurden 2-mal mit einem Parker-TC-199-Medium gewaschen und im gleichen Medium suspendiert ($10^6$ Zellen/ml), die Zellensuspension wurde in einem dunklen Behälter 15 Minuten lang stehengelassen.

Es wurde 3-(Amino)-phthalsäurehydrazid [Luminol] in einer konzentrierten Ammoniumhydroxydlösung gelöst und mit einer Lösung der folgenden Zusammensetzung verdünnt:
50 ml Tris-Parker-Medium (enthaltend 1,2 g Tris-(hydroxymethyl)-aminomethan [Tris], pH-Wert: 7,4)
150 ml Parker-Gewebekulturmedium
5 ml Glucose, 40 Vol.-% in Wasser.

Die Endkonzentration des 3-(Amino)-phthalsäurehydrazides [Luminoles] betrug 32 µM. Die Lösung des 3-(Amino)-phthalsäurehydrazides [Luminoles] (500 µl) wurde in Kunststoffampullen eingewogen und im Dunkeln gehalten (bei 37°C). Die Reaktion wurde durch Zugabe der Zellen und des stimulierenden Mittels (Phythämagglutinin [PHA], 10 µm/ml) induziert.

Die Ampullen wurden kontinuierlich leicht geschüttelt und die Phythämagglutinin-Stimulierung [PHA-Stimulierung] alle 2,5 Minuten während 15 Minuten in einem Spektrometer (Beckmann LS-100) gemessen (Koinzidenzkreis ausgeschaltet). Die prozentualen Werte der Zählungen wurden mit den entsprechenden Werten der mit dem Lösungsmittel behandelten Blindversuchs- beziehungsweise Kontrollzellen verglichen. Für jede untersuchte Verbindung wurden mindestens 5 Konzentrationen gemessen und mindestens 3 parallele Bestimmungen durchgeführt. Die Ergebnisse sind in der folgenden Tabelle IV zusammengestellt.

Tabelle IV

| Chemilumineszenz von phythämagglutinin-stimulierten [PHA-stimulierten] Granulocyten | | |
|---|---|---|
| Untersuchte Verbindung | | $IC_{50}$-Wert in Mol |
| Bezeichnung | Beispiel Nr. | |
| 2,4-Di-[amino]-5-[4′-(methoxy)-benzyl]-pyrimidin | 1 | etwa $10^{-4}$ |
| 2,4-Di-[amino]-5-[3′-(methoxy)-benzyl]-pyrimidin | 2 | etwa $10^{-4}$ |
| 2,4-Di-[amino]-5-[3′-(methoxy)-4′-(äthoxy)-benzyl]-pyrimidin | 7 | etwa $10^{-5}$ |
| 2,4-Di-[amino]-5-[3′-(methoxy)-4′-allyloxy)-benzyl]-pyrimidin-hydrochlorid | 8 | etwa $10^{-6}$ |
| 2,4-Di-[amino]-5-[3′-(methoxy)-4′-(n-butoxy)-benzyl]-pyrimidin | 9 | etwa $10^{-6}$ |

Fortsetzung der Tabelle IV

| Untersuchte Verbindung | | $IC_{50}$-Wert in Mol |
|---|---|---|
| Bezeichnung | Beispiel Nr. | |
| 1-[4′-(Chlor)-benzoyl]-5-[methoxy]-2-[methyl]-indol-3-ylessigsäure [Indometacin] | Vergleichssubstanz A | $10^{-4}$ |
| 4-(n-Butyl)-1,2-di-(phenyl)-pyrazolidin-3,5-dion [Phenylbutazon] | Vergleichssubstanz C | $10^{-4}$ |
| 4-(Hydroxy)-2-(methyl)-N-(2′-pyridyl)-2H-1,2-benzothiazin-3-carboxamid-1,1-di-(oxyd) [Piroxicam] | Vergleichssubstanz D | $10^{-4}$ |

Die 2,4-Di-(amino)-pyrimidinderivate hemmen das chemisch induzierte Erscheinen der freien Radikale. Die Wirkung dieser Verbindungen ist um bis zu mehreren Größenordnungen höher als die der verwendeten bekannten Antiphlogistica. In mononuklearen Zellen beträgt das $IC_{50}$-Volumen dieser Verbindungen 3 . $10^{-5}$ M. 4-(Hydroxy)-2-(methyl)-N-(2′-pyridyl)-2H-1,2-benzothiazin-3-carboxamid-1,1-di-(oxyd) [Piroxicam] und 4-(n-Butyl)-1,2-di-(phenyl)-pyrazolidin-3,5-dion [Phenylbutazon] haben sich an diesen Zellen als unwirksam erwiesen. Die 2,4-Di-(amino)-pyrimidinderivate beeinflussen die durch Wasserstoffperoxyd hervorgerufene Chemilumineszenz in zellenfreiem Medium nicht.

## Schlußfolgerungen

Es ist auf Grund der Messungen der Chemilumineszenz festzustellen, daß die 2,4-Di-(amino)-pyrimidinderivate die Bildung der freien Sauerstoff-Radikale hemmen. Diese freien Radikale verursachen Gewebeschädigungen und die Hemmung der Bildung der obigen freien Radikale ist therapeutisch wertvoll und günstig. Die die obige Hemmwirkung aufweisenden Verbindungen können als Antiphlogistica Verwendung finden und den Alterungsvorgang hemmen. Es ist nämlich bekannt, daß beim Vorgang des Alterns die durch freie Sauerstoff-Radikale verursachten Schädigungen der strukturellen Elemente eine wichtige Rolle spielen.

Die obigen Prüfversuche wurden nach an sich bekannten Verfahrensweisen durchgeführt. Die einschlägigen Schrifttumsstellen sind nachstehend zusammengefaßt:

Litchfield, J. T., Wilcoxon, F.: J. Pharmacol. Exp. Ther. 96 [1949], 99 bis 113;
Newbould, B. B.: Brit. J. Pharmacol. 35 [1969], 487;
Shay, H., Komarov, S. A., Fels, S. S., Meranze, D., Gruenstein, M., Siplet, H.: Gastroenterology 5 [1945], 45;
Stickney, J. C., Northup, D. W., Van Liere, E. J.: Arch. int. Pharmacodyn. 147 [1964], 113;
Winter, C. A., Risley, E. A., Nuss, G. W.: Proc. Soc. Exp. Biol. Med. 111 [1962], 544 bis 547.

Ein besonderer Vorteil der 2,4-Di-(amino)-pyrimidinderivate der allgemeinen Formel I beziehungsweise ihrer Säureadditionssalze besteht darin, daß diese nicht nur die analgetische und entzündungshemmende Wirkung der auf diesem Gebiet verwendeten Handelsprodukte wesentlich übertreffen, sondern auch nicht für die bekannten entzündungshemmenden und analgetischen Arzneimittel charakteristische, die Magenschleimhaut schädigende Nebenwirkung zeigen. Dies bedeutet, daß diese Verbindungen die gewünschte analgetische und entzündungshemmende Wirkung ohne ulcerogene Nebenwirkungen entfalten.

Die 2,4-Di-(amino)-pyrimidinderivate können in Form von Arzneimittelpräparaten vorliegen. Die letzteren können nach an sich bekannten Verfahrensweisen der pharmazeutischen Technik in der Weise hergestellt werden, daß 2,4-Di-(amino)-pyrimidinderivate der allgemeinen Formel I oder deren Säureadditionssalze mit inerten festen und/oder flüssigen pharmazeutischen Trägern und/oder Hilfsstoffen vermischt und in eine galenische Form gebracht werden.

Zweckmäßig liegen die 2,4-Di-(amino)-pyrimidinderivate in Zubereitungsformen zur oralen, rektalen oder parenteralen Verabreichung vor.

Die Präparate zur oralen Verabreichung können vorteilhaft Tabletten, Dragees, Kapseln, darmlösliche [enterosolvente] Tabletten, Lösungen oder Suspensionen sein; weitere vorteilhafte Zubereitungsformen sind Suppositorien und Injektionspräparate. Der Wirkstoffgehalt solcher Arzneimittelpräparate beträgt etwa 10 bis etwa 500 mg.

Die oral angewendeten Arzneimittelpräparate können übliche Träger und/oder Hilfsstoffe, zum Beispiel Milchzucker [Lactose], Stärke, Magnesiumstearat, Natriumcitrat, Calciumcarbonat, Dicalciumphosphat, Stärkederivate, wie Carboxymethylstärke, Kieselsäure und/oder Bindemittel, wie Polyvinylpyrrolidon, enthalten. Die Tabletten können ferner Gleitmittel, wie Magnesiumstearat und/oder Talk, enthalten.

Die Tabletten können nach üblichen trockenen oder feuchten Granulierverfahren hergestellt werden. Bei der Herstellung von Dragees wird der Drageekern auf übliche Weise mit einem Überzug versehen. Die Kapseln werden in der Weise hergestellt, daß die Zubereitungsmischung in Hart- oder Weichgelatinekapseln gefüllt wird.

Zur oralen Verabreichung sind auch wäßrige Suspensionen und/oder Elixiere geeignet. Diese Präparate können übliche Verdünnungsmittel, zum Beispiel Wasser, Äthanol, Propylenglykol und/oder Glycerin, und daneben übliche Zusatzstoffe, zum Beispiel geschmackverbessernde Mittel, Farbstoffe, Emulgiermittel und/oder Stabilisiermittel, wie Methyl-p-(hydroxy)-benzoat, enthalten.

Zweckmäßig beträgt der Wirkstoffgehalt der zur rektalen Verabreichung geeigneten Suppositorien etwa 0,01 bis 0,5 g. Die Suppositorien können in der Weise hergestellt werden, daß der beziehungsweise die Wirkstoff(e) in der geschmolzenen Suppositoriengrundmasse, zum Beispiel Kakaobutter oder Witepsol ®

H15, gleichmäßig verteilt und die Masse in geeignete Formen gefüllt, abgekühlt und in Aluminium- oder Stanniolfolien verpackt wird.

Die zur parenteralen Verabreichung geeigneten Injektionslösungen können intramuskulär, intraperitoneal oder subkutan verabreicht werden. Der Wirkstoffgehalt der Injektionslösungen beträgt zweckmäßig etwa 0,005 bis 0,25 g/ml. Die Injektionslösungen werden üblicherweise in 1 ml oder 2 ml Ampullen gefüllt; der Wirkstoffgehalt der Ampullen beträgt im allgemeinen 0,0025 bis 0,25 g/Ampulle. Die parenteral verabreichbaren Injektionslösungen können als Verdünnungsmittel zum Beispiel Wasser, Sesamöl, Erdnußöl, wäßriges Propylenglykol und/oder andere pharmazeutisch brauchbare Lösungsmittel enthalten. Die wäßrigen Lösungen haben sich als besonders vorteilhaft erwiesen. In den Injektionslösungen liegen die 2,4-Di-(amino)-pyrimidinderivate der allgemeinen Formel vorzugsweise in Form von wasserlöslichen Säureadditionssalzen vor. Die wäßrigen Lösungen können erforderlichenfalls auf übliche Weise gepuffert oder durch Zugabe von Natriumchlorid oder Glucose isotonisch gemacht werden. Die erhaltenen Lösungen können gegebenenfalls in an sich bekannter Weise sterilisiert werden.

Die tägliche Dosis der 2,4-Di-(amino)-pyrimidinderivate der allgemeinen Formel I beziehungsweise von deren Säureadditionssalzen kann innerhalb weiter Grenzen variiert werden. Erwachsenen kann beziehungsweise können zur Linderung von leichten bis mittelmäßigen Schmerzen, zum Beispiel Kopfschmerz, Zahnschmerz, Kreuzweh, Lumbago, Neuralgie, Myalgie, fieberhaften Erkältungskrankheiten und postoperativen Schmerzen, täglich 2 bis 3-mal 1 oder 2 Tablette(n) mit einem Wirkstoffgehalt von 10 bis 500 mg verabreicht werden.

Zur Behandlung von chronischen rheumatischen Entzündungen und degenerativem Rheumatismus können täglich 8 bis 10 Tabletten verabreicht werden, und zwar alle 6 bis 8 Stunden je 2 Tabletten.

Die für Kinder zu empfehlende tägliche Dosis beträgt 3-mal oder 4-mal $\frac{1}{2}$ oder 1 Tablette mit einem Wirkstoffgehalt von 10 bis 250 mg.

Die tägliche Dosis der 2,4-Di-(amino)-pyrimidinderivate kann bei verschiedenen Indikationen etwa 5 mg bis etwa 4 000 mg betragen.

Bei Verwendung der 2,4-Di-(amino)-pyrimidinderivate der allgemeinen Formel I oder von deren Säureadditionssalzen als analgetischen oder antipyretischen Mitteln beträgt die optimale Dosis etwa 5 bis 2 000 mg und zwar auf 3 bis 4 Portionen verteilt. Für Kinder unter 11 Jahren beträgt die tägliche Dosis vorzugsweise 5 bis 250 mg und für Erwachsene 50 bis 2 000 mg.

Zur Behandlung von rheumatischen Entzündungen und rheumatischen Krankheiten verschiedenen Ursprunges (degenerative beziehungsweise nicht-arthritische Krankheiten) beträgt die tägliche Dosis für Kinder vorzugsweise 100 bis 1 500 mg und für Erwachsene etwa 300 bis 4 000 mg.

Die obigen Bereiche sind nur informativer Art und die tatsächlich zu verabreichende Dosis wird immer unter Berücksichtigung sämtlicher Bedingungen des gegebenen Falles und auf Grund der Vorschläge des Arztes bestimmt.

Die 2,4-Di-(amino)-pyrimidinderivate der allgemeinen Formel I und deren Säureadditionssalze können nach an sich bekannten Verfahren hergestellt werden. So können sie zum Beispiel in der Weise hergestellt werden, daß

a) substituierte β-(Cyan)-β-(methoxymethyl)-styrole der allgemeinen Formel

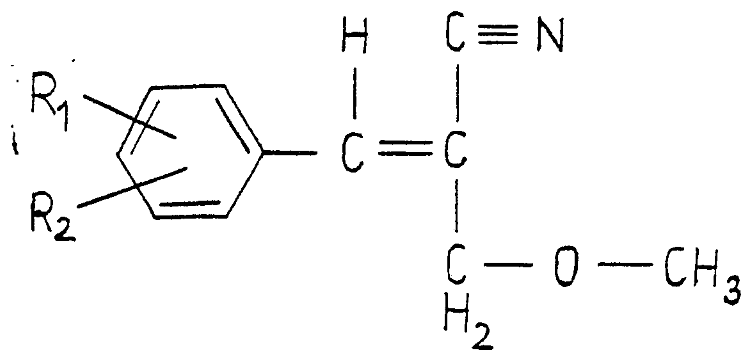

II ,

worin

$R_1$ und $R_2$ die obigen Bedeutungen haben,

mit Diäthylenglykol-mono-(niederalkyl)-äthern der allgemeinen Formel

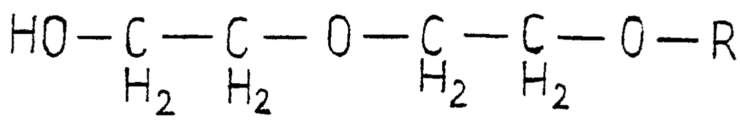

III ,

worin
R für einen Alkylrest mit 1 bis 5, vorzugsweise 1 bis 4, Kohlenstoffatom(en) steht, umgesetzt werden und die so erhaltenen substitu ierten 1-(Phenyl)-2-(cyan)-3-(ω-{alkoxy}-di-[äthoxy])-prop-3-ene der allgemeinen Formel

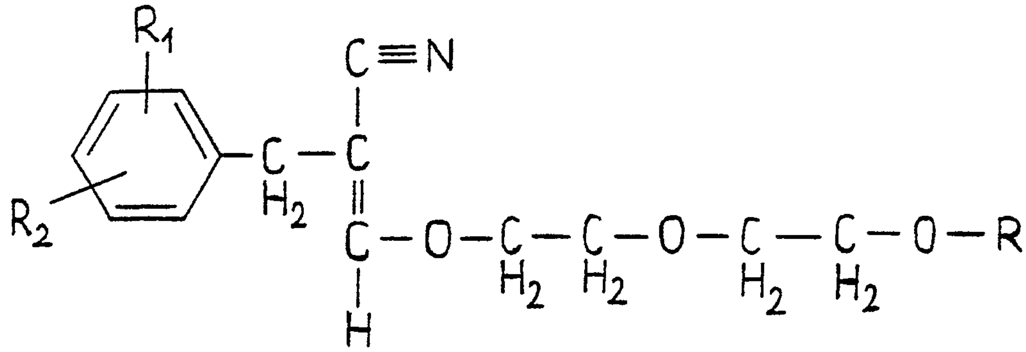

IV ,

worin
R, $R_1$ und $R_2$ die obigen Bedeutungen haben,
mit Guanidin oder dessen Säureadditionssalzen umgesetzt werden oder

b) substituierte β-(Cyan)-β-(methoxymethyl)-styrole der allgemeinen Formel II mit Äthylenglykol-mono-(niederalkyl)-äthern der allgemeinen Formel

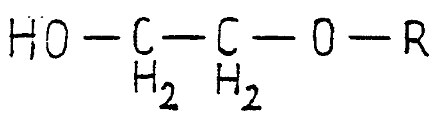

V ,

worin
R die obige Bedeutung hat,
umgesetzt werden und die so erhaltenen substituierten 1-(Phenyl)-2-(cyan-3-(ω-{alkoxy}-äthoxy)-prop-3-ene der allgemeinen Formel

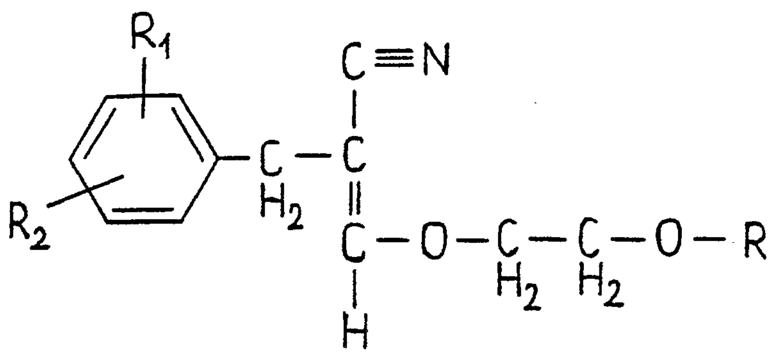

VI ,

worin
R, $R_1$ und $R_2$ die obigen Bedeutungen haben,
mit Guanidin oder dessen Säureadditionssalzen umgesetzt werden und gegebenenfalls in den erhaltenen 5-(substituierten Benzyl) 2,4-Di-(amino)-pyrimidinderivaten der allgemeinen Formel I [ein] Rest(e), für welche[n] $R_1$ und/oder $R_2$ stehen kann beziehungsweise können, in [einen] andere[n] umgewandelt wird beziehungsweise werden und/oder

gegebenenfalls die erhaltenen 5-(substituierten Benzyl)2,4-Di-(amino)-pyrimidinderivate der allgemeinen Formel I in Säureadditionssalze überführt und/oder die erhaltenen Säureadditionssalze der 5-(substituierten Benzyl) 2,4-Di-(amino)-pyrimidinderivate der allgemeinen Formel I in die freien 5-(substituierten Benzyl) 2,4-Di-(amino)-pyrimidinderivatbasen und/oder andere Säureadditionssalze überführt werden.

Die als Ausgangssubstanzen eingesetzten substituierten β-(Cyan)-β-(methoxymethyl)-styrole der allgemeinen Formel II können durch Umsetzen von substituierten Benzaldehyden der allgemeinen Formel

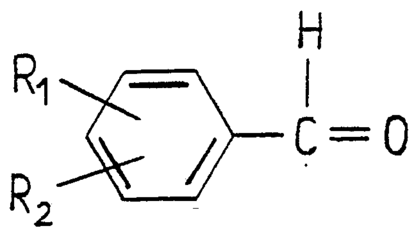

VII ,

worin

$R_1$ und $R_2$ die obigen Bedeutungen haben,

mit 3-(Methoxy)-propionitril der Formel

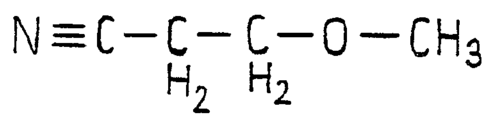

VIII

hergestellt werden. Die erhaltenen substituierten β-(Cyan)-β-(methoxymethyl)-styrole können auch ohne deren Isolieren beziehungsweise Reinigen weiterumgesetzt werden.

Die Erfindung wird an Hand der folgenden Beispiele näher erläutert.

I.) Herstellung von 2,4-Di-(amino)-pyrimidinderivaten der allgemeinen Formel I beziehungsweise ihren Säureadditionssalzen

## Beispiel 1

### 2,4-Di-[amino]-5-[4′-(methoxy)-benzyl]-pyrimidin

Es wurde ein Gemisch von 27,2 g (0,2 Mol) 4-(Methoxy)-benzaldehyd (Fluka® 10440), 25,5 g 3-(Methoxy)-propionitril, 70 ml Methanol und 3 g Kaliumhydroxyd 8 Stunden lang bei 60 °C gerührt beziehungsweise geschüttelt. Das Reaktionsgemisch wurde abgekühlt, mit 500 ml Wasser verdünnt und 2-mal mit je 100 ml Benzol extrahiert. Die organische Phase wurde getrocknet und unter vermindertem Druck eingedampft. Der Rückstand wurde in 50 ml Diäthylenglykolmonomethyläther gelöst. Nach Zugabe von 3 g pulverförmigem Natriummethylat wurde das Reaktionsgemisch auf 75 bis 77 °C erwärmt und 3 Stunden lang bei dieser Temperatur gerührt beziehungsweise geschüttelt. Zum Gemisch wurden 80 ml Isobutanol, 43 g Guanidinhydrochlorid und eine 30 gew.-%-ige methanolische Lösung von 27 g Natriummethylat zugegeben. Das Reaktionsgemisch wurde auf 92 °C erwärmt, wobei Methanol abdestilliert wurde. Die Umsetzung wurde 7 Stunden lang bei dieser Temperatur fortgesetzt. Nach dem Abkühlen wurde das auskristallisierte Produkt abfiltriert und aus wäßrigem Alkohol umkristallisiert. So wurden 23,2 g 2,4-Di-[amino]-5-[4′-(methoxy)-benzyl]-pyrimidin erhalten. Ausbeute: 57% der Theorie; F.: 206 bis 209 °C.
Analyse: auf die Formel $C_{12}H_{14}N_4O$
berechnet : C% = 62,59; H% = 6.13; N% = 24,33;
gefunden : C% - 62,61; H% = 5,96; N% = 24,21.

## Beispiele 2 bis 7

In analoger Weise wie im Beispiel 1 wurden die folgenden Verbindungen hergestellt.

## Beispiel 2

2,4-Di-[amino]-5-[3′-(methoxy)-benzyl]-pyrimidin

Diese Verbindung wurde aus 3-(Methoxy)-benzaldehyd (Fluka ® 64780) hergestellt. F.: 221 bis 223 °C.
Analyse: auf die Formel $C_{12}H_{14}N_4O$
berechnet : C% =62,59; H% = 6,13; N% = 24,33;
gefunden : C% =62,58; H% = 6,05; N% = 24,21.

## Beispiel 3

2,4-Di-[amino]-5-[3′,5′-di-(methoxy)-benzyl]-pyrimidin

Diese Verbindung wurde aus 3,5-Di-(methoxy)-benzaldehyd (Fluka ® 38630) hergestellt. F.: 164 bis 168 °C.
Analyse: auf die Formel $C_{13}H_{16}N_4O_2$
berechnet: C% = 59,98; H% = 6,19; N% = 21,56;
gefunden : C% = 60,32; H% = 6,26; N% = 21,65.

## Beispiel 4

2,4-Di-[amino]-5-[2′,4′-di-(methoxy)-benzyl]-pyrimidin

Diese Verbindung wurde aus 2,4-Di-(methoxy)-benzaldehyd (Fluka ® 38619) hergestellt. F.: 170 bis 174 °C.
Analyse: auf die Formel $C_{13}H_{16}N_4Q_2$
berechnet: C% = 59,98; H% = 6,19; N% = 21,56;
gefunden : C% = 60,02; H% = 6,29; N% = 21,35.

## Beispiel 5

2,4-Di-[amino]-5-[2′,3′-di-(methoxy)-benzyl]-pyrimidin

Diese Verbindung wurde aus 2,3-Di-(methoxy)-benzaldehyd (Fluka ® 38610 hergestellt. F.: 194 bis 198° C.
Analyse: auf die Formel $C_{13}H_{16}N_4O_2$
berechnet : C% = 59,98; H% = 6,19; N% = 21,56;
gefunden ; C% = 59,97; H% = 6,27; N% = 21,47.

Beispiel 6

2,4-Di-[amino]-5-[3′,4′-(methylendioxy)-benzyl]-pyrimidin

Diese Verbindung wurde aus 3,4-(Methylendioxy)-benzaldehyd (Fluka ® 80620 hergestellt. F.: 248 bis 255° C.
Analyse: auf die Formel $C_{12}H_{12}H_2O_2$
berechnet: C% = 59,01; H% = 4,95; N% = 22,94;
gefunden : C% = 59,45; H% = 5,14; N% = 22,66.

Beispiel 7

2,4-Di-[amino]-5-[3′-(methoxy)-4′-(äthoxy)-benzyl]-pyrimidin

Diese Verbindung wurde aus 3-(Methoxy)-4-(äthoxy)-benzaldehyd hergestellt. F.: 195 bis 196° C.
Analyse: auf die Formel $C_{14}H_{18}N_4O_2$
berechnet: C% = 61,29; H% = 6,61; N% = 20,42;
gefunden : C% = 60,84; H% = 6,58; N% = 20,0.

Der als Ausgangssubstanz verwendete 3-(Methoxy)-4-(äthoxy)-benzaldehyd wurde wie folgt hergestellt.

Es wurden zu einer Lösung von 30,4 g (0,2 Mol) Vanillin, 12 g (0,3 Mol) Natriumhydroxyd und 300 ml Wasser 37,4 g (0,24 Mol) Äthyljodid zugegeben. Das Reaktionsgemisch wurde 15 Stunden lang zum Sieden erhitzt. Nach dem Abkühlen wurde das Produkt mit Benzol extrahiert und der Extrakt eingedampft. Der rohe Rückstand wurde in 110 ml einer 25 Gew.-%-igen Natriumbisulfitlösung gelöst, mit Aktivkohle geklärt und auf den pH-Wert von 12 alkalisch gemacht. Das ausgeschiedene kristalline Produkt wurde abfiltriert, mit Wasser gründlich gewashcen und getrocknet. So wurden 24,8 g 3-(Methoxy)-4-(äthoxy)-benzaldehyd erhalten. Ausbeute: 69% der Theorie; F.: 65 bis 67° C.

Beispiel 8

2,4-Di-[amino]-5-[3′-(methoxy)-4′-(allyloxy)-benzyl]-pyrimidin-hydrochlorid

Diese Verbindung wurde analog wie im Beispiel 1 beschrieben aus 3-(Methoxy)-4-(allyloxy)-benzaldehyd hergestellt. Das Rohprodukt wurde in wäßriger Salzsäure gelöst, mit Aktivkohle geklärt und eingedampft. Das ausgeschiedene kristalline Produkt wurde abfiltriert, mit wäßrigem Alkohol gewaschen und

getrocknet. Das so erhaltene 2,4-Di-[amino]-5-[3′-(methoxy)-4′-(allyloxy)-benzyl]-pyrimidin-hydrochlorid hatte einen Schmelzpunkt von 236 bis 237 °C.
Analyse: auf die Formel $C_{15}H_{18}N_4O_2$.HCl
berechnet: C% = 55,86; H% = 5,93, N% = 17,37; Cl% = 11,43;
gefunden : C% = 55,73; H% = 5,87, N% = 17,21; Cl% = 11,30.

Der als Ausgangssubstanz verwendete 3-(Methoxy)-4-(allyloxy)-benzaldehyd wurde analog wie für die Ausgangssubstanz des Beispieles 7 beschrieben unter Verwendung von Allylbromid hergestellt.
Der Brechungsindex des erhaltenen hellgelben Öles betrug $n_D^{25}$ = 1,5772.

## Beispiel 9

### 2,4-Di-[amino]-5-[3′-(methoxy)-4′-(n-butoxy)-benzyl]-pyrimidin

Diese Verbindung wurde aus 3-(Methoxy)-4-(n-butoxy)-benzaldehyd analog wie im Beispiel 1 beschrieben hergestellt. F.: 143 bis 147 °C.
Analyse: auf die Formel $C_{16}H_{22}N_4O_2$
berechnet: C% = 63,55; H% = 7,33; N% = 18,53;
gefunden : C% = 63,12; H% = 7,48; N% = 18,40.

Der als Ausgangsstoff verwendete 3-(Methoxy)-4-(n-butoxy)-benzaldehyd wurde analog wie für die Ausgangssubstanz des Beispieles 7 beschrieben unter Verwendung von n-Butylbromid hergestellt.
Der Brechungsindex des erhaltenen hellgelben Öles betrug $n_D^{25}$ = 1,5535.

## Beispiel 10

### 2,4-Di-[amino]-5-[3′-(methoxy)-4′-(2″-{methoxy}-äthoxy)-benzyl]-pyrimidin

Diese Verbindung wurde aus 3-(Methoxy)-4-(2′-{methoxy}-äthoxy)-benzaldehyd analog wie im Beispiel 1 beschrieben hergestellt. F.: 163 bis 164 °C.
Analyse: auf die Formel $C_{15}H_{20}N_4O_3$
berechnet : C% = 59,19; H% = 6,62; N% = 18,41;
gefunden ; C% = 58,80; H% = 6,59; N% = 18,42.

Der als Ausgangssubstanz 3-(Methoxy)-4-(2′-{methoxy}-äthoxy)-benzaldehyd wurde analog wie für die Ausgangssubstanz des Beispieles 7 beschrieben hergestellt. F.: 64 bis 67 °C.

## Beispiel 11

### 2,4-Di-[amino]-5-[3′-(methoxy)-4′-(benzyloxy)-benzyl]-pyrimidin

Diese Verbindung wurde aus 3-(Methoxy)-4-(benzyloxy)benzaldehyd analog wie im Beispiel 1 beschrieben hergestellt. F.: 198 bis 202 °C.
Analyse: auf die Formel $C_{19}H_{20}N_4O_2$

berechnet: C% = 67,84; H% = 5,99; N% = 16,65;
gefunden : C% = 67,40; H% = 5,98; N% = 16,35.

Der als Ausgangssubstanz verwendete 3-(Methoxy)-4-(benzyloxy)-benzaldehyd wurde analog wie für die Ausgangssubstanz des Beispieles 7 beschrieben hergestellt. F.: 59 bis 63 ° c.

Beispiel 12

2,4-Di-[amino]-5-[3′-(methoxy)-4′-(hydroxy)-benzyl]-pyrimidin-hydrobromid

Es wurden 10 g wie im Beispiel 11 beschrieben hergestelltes 2,4-Di-[amino]-5-[3′-(methoxy)-4′-(benzyloxy)-benzyl]-pyrimidin in 500 ml Dioxan gelöst. Es wurde ein Hydrieren in Gegenwart von 0,2 g eines Palladium/Kohle-Katalysators unter einem Druck von 0,3 MPa (3 A tm) bei 60 bis 65 ° C durchgeführt. Nach Aufnahme der berechneten Wasserstoffmenge wurde der Katalysator abfiltriert und das Filtrat unter vermindertem Druck eingedampft. Der Rückstand wurde in einer Mischung von 50 ml Wasser und 8 g einer 48 Gew.-%-igen Bromwasserstofflösung gelöst. Nach dem Abkühlen wurde das auskristallisierte Produkt abfiltriert und getrocknet. So wurden 7,9 g 2,4-Di-[amino]-5-[3′-(methoxy)-4′-(hydroxy)-benzyl]-pyrimidin-hydrobromid erhalten. F.: 261 bis 263 ° C.
Analyse: auf die Formel $C_{12}H_{14}N_4O_2.HBr$
berechnet: C% = 44,05; H% = 4,62; N% = 17,12; Br% = 24,44;
gefunden : C% = 44,12; H% = 4,63; N% = 16,66; Br% = 23,86.

II.) Herstellung von Arzneimittelpräparaten

Beispiel 13

Es wurden Tabletten der folgenden Zusammensetzung nach an sich bekannten Verfahrensweisen der pharmazeutischen Technik hergestellt:

| Bestandteil | Menge in g/Tablette |
|---|---|
| 2,4-Di-[amino]-5-[4′-(methoxy)-benzyl]-pyrimidin nach Beispiel 1 | 0,200 |
| Milchzucker | 0,110 |
| Kartoffelstärke | 0,055 |
| Natriumamylopektinglykolat | 0,010 |
| Gelatine | 0,008 |
| Magnesiumstearat | 0,001 |
| Gesamtgewicht | 0,384 g |

Beispiel 14

Es wurden Tabletten der folgenden Zusammensetzung nach an sich bekannten Verfahrensweisen der pharmazeutischen Technik hergestellt:

| Bestandteil | Menge in g/Tablette |
|---|---|
| 2,4-Di-[amino]-5-[3′-(methoxy)-benzyl]-pyrimidin nach Beispiel 2 | 0,400 |
| Milchzucker | 0,150 |
| Kartoffelstärke | 0,080 |
| Natriumamylopektinglykolat | 0,020 |
| Gelatine | 0,016 |
| Magnesiumstearat | 0,002 |
| Gesamtgewicht | 0,668 g |

## Beispiel 15

Es wurden Suppositorien der folgenden Zusammensetzung nach an sich bekannten Verfahrensweisen der pharmazeutischen Technik hergestellt:

| Bestandteil | Menge in g/Suppositorium |
|---|---|
| 2,4-Di-[amino]-5-[3′,5′-di-(methoxy)-benzyl]-pyrimidin nach Beispiel 3 | 0,160 |
| Witepsol® H 15 | 1,340 |
| | 1,500 |

## Beispiel 16

Es wurden Suppositorien der folgenden Zusammensetzung nach an sich bekannten Verfahrensweisen der pharmazeutischen Technik hergestellt:

| Bestandteil | Menge in g/Suppositorium |
|---|---|
| 2,4-Di-[amino]-5-[2′,4′-di-(methoxy)-benzyl]-pyrimidin nach Beispiel 4 | 0,250 |
| Witepsol H 15 | 1,500 |
| | 1,750 |

## Beispiel 17

Es wurden Injektionslösungen nach an sich bekannten Verfahrensweisen der pharmazeutischen Technik hergestellt:

| Bestandteil | Menge |
|---|---|
| 2,4-Di-[amino]-5-[3′-(methoxy)-4′-(allyloxy)-benzyl]-pyrimidin-hydrochlorid nach Beispiel 8 | 0,100 g |
| Ascorbinsäure | 0,187 g |
| Destilliertes Wasser zum Auffüllen auf | 2,0 ml |

Beispiel 18

Es wurden Injektionslösungen nach an sich bekannten Verfahrensweisen der pharmazeutischen Technik hergestellt:

| Bestandteil | Menge |
|---|---|
| 2,4-Di-[amino]-5-[3′-(methoxy)-4′-(allyloxy)-benzyl]-pyrimidin-hydrochlorid nach Beispiel 8 | 0,200 g |
| Ascorbinsäure | 0,374 g |
| Destilliertes Wasser zum Auffüllen auf | 5,0 ml |

Beispiel 19

Es wurde eine Suspension der folgenden Zusammensetzung nach an sich bekannten Verfahrensweisen der pharmazeutischen Technik hergestellt:

| Bestandteil | Menge |
|---|---|
| 2,4-Di-[amino]-5-[3′-(methoxy)-4′-(2″-{methoxy}-äthoxy)-benzyl]-pyrimidin nach Beispiel 10 | 2,0 g |
| Methyl-p-(hydroxy)-benzoat | 0,1 g |
| Spiritus anisatus | 0,25 g |
| Xanthangummi [Keltrol] | 61,0 g |
| Destilliertes Wasser zum Auffüllen auf | 100,0 ml |

**Ansprüche**

1.) Verwendung von 5-(substituierten Benzyl) 2,4-Di-(amino)-pyrimidinderivaten der allgemeinen Formel

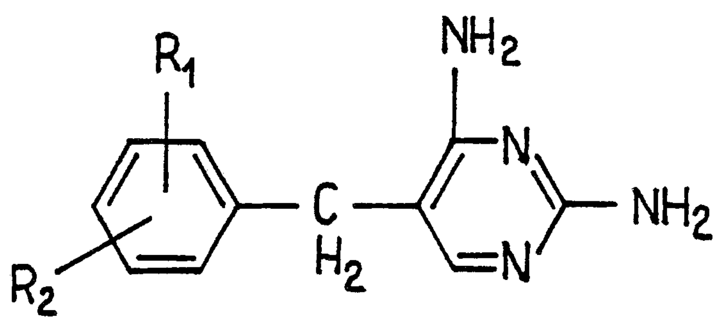

I ,

worin

$R_1$ und $R_2$ , die gleich oder verschieden sein können, für Wasserstoffatome, Hydroxygruppen, Alkoxyreste mit 1 bis 6 Kohlenstoffatom(en), Alkoxyalkoxyreste mit 1 bis 6 Kohlenstoffatom(en) in jedem Alkoxyteil, Alkenyloxyreste mit 2 bis 6 Kohlenstoffatomen beziehungsweise Phenylalkoxyreste mit 1 bis 3 Kohlenstoffatom(en) im Alkoxyteil stehen oder

$R_1$ und $R_2$ zusammen einen Alkylendioxyrest mit 1 oder 2 Kohlenstoffatom(en) darstellen,

mit der weiteren Maßgabe, daß

mindestens 1 von

$R_1$ und $R_2$ von Wasserstoff verschieden ist,

sowie ihre Säureadditionssalze zur Herstellung von antipyretisch; entzündungshemmend einschließlich

antirheumatisch, anti-anginös und/oder antioxydierend wirkenden Arzneimitteln.

2.) 5-(Substituierte Benzyl) 2,4-Di-(amino)-pyrimidinderivate der allgemeinen Formel

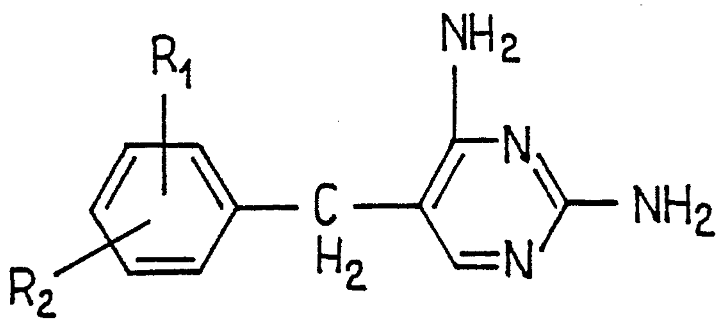

I ,

worin

$R_1$ und $R_2$ , die gleich oder verschieden sein können, für Wasserstoffatome, Hydroxygruppen, Alkoxyreste mit 1 bis 6 Kohlenstoffatom(en), Alkoxyalkoxyreste mit 1 bis 6 Kohlenstoffatom(en) in jedem Alkoxyteil, Alkenyloxyreste mit 2 bis 6 Kohlenstoffatomen beziehungsweise Phenylalkoxyreste mit 1 bis 3 Kohlenstoffatom(en) im Alkoxyteil stehen oder

$R_1$ und $R_2$ zusammen einen Äthylendioxyrest darstellen,

mit der weiteren Maßgabe, daß

im Falle daß

das eine von $R_1$ und $R_2$ für Wasserstoff oder einen Alkoxy- oder Benzyloxyrest steht,

das andere von $R_1$ und $R_2$ von Wasserstoff oder einem Alkoxy- oder Benzyloxyrest verschieden ist,

sowie ihre Säureadditionssalze, insbesondere zur Verwendung als Arzneimittel.

3.) Verwendung nach Anspruch 1 oder 2,4-Di-(amino)-pyrimidinderivate nach Anspruch 2, dadurch gekennzeichnet, daß die 2,4-Di-(amino)-pyrimidinderivate solche, bei welchen der beziehungsweise die Alkoxyrest(e), für welche[n] $R_1$ und/oder $R_2$ stehen kann beziehungsweise können, [ein] solche[r] mit 1 bis 4, insbesondere 1 oder 2, Kohlenstoffatom(en) ist beziehungsweise sind, sind.

4.) Verwendung nach Anspruch 1 oder 3 oder 2,4-Di-(amino)-pyrimidinderivate nach Anspruch 2 oder 3, dadurch gekennzeichnet, daß die 2,4-Di-(amino)-pyrimidinderivate solche, bei welchen der beziehungsweise die Alkoxyalkoxyrest(e), für welche[n] $R_1$ und/oder $R_2$ stehen kann beziehungsweise können, [ein] solche[r] mit 1 bis 4, insbesondere 1 und/oder 2, Kohlenstoffatom(en) in jedem Alkoxyteil ist beziehungsweise sind, sind.

5.) Verwendung nach Anspruch 1, 3 oder 4 oder 2,4-Di-(amino)-pyrimidinderivate nach Anspruch 2 bis 4, da durch gekennzeichnet, daß die 2,4-Di-(amino)-pyrimidinderivate solche, bei welchen der beziehungsweise die Alkenyloxyrest(e), für welche[n] $R_1$ und/oder $R_2$ stehen kann beziehungsweise können, [ein] solche[r] mit 2 bis 4, insbesondere 2 oder 3, Kohlenstoffatomen ist beziehungsweise sind, sind.

6.) Verwendung nach Anspruch 1 oder 3 bis 5 oder 2,4-Di-(amino)-pyrimidinderivate nach Anspruch 2 bis 5, dadurch gekennzeichnet, daß die 2,4-Di-(amino)-pyrimidinderivate solche, bei welchen der beziehungsweise die Phenylalkoxyrest(e), für welche[n] $R_1$ und/oder $R_2$ stehen kann beziehungsweise können, [ein] solche[r] mit 1 oder 2 Kohlenstoffatom(en) im Alkoxyteil ist beziehungsweise sind, sind.

7.) Verwendung nach Anspruch 1 oder 3 bis 6 oder 2,4-Di-(amino)-pyrimidinderivate nach Anspruch 2 bis 6, dadurch gekennzeichnet, daß die Säureadditionssalze der 2,4-Di-(amino)-pyrimidinderivate der allgemeinen Formel I Hydrochloride sind.

8.) Verwendung nach Anspruch 1 oder 3 bis 7 oder 2,4-Di-(amino)-pyrimidinderivate nach Anspruch 2 bis 7, dadurch gekennzeichnet, daß die 2,4-Di-(amino)-pyrimidinderivate

2,4-Di-[amino]-5-[4′-(methoxy)-benzyl]-pyrimidin,

2,4-Di-[amino]-5-[3′-(methoxy)-benzyl]-pyrimidin,

2,4-Di-[amino]-5-[3′,5′-di-(methoxy)-benzyl]-pyrimidin,

2,4-Di-[amino]-5-[2′,4′-di-(methoxy)-benzyl]-pyrimidin,

2,4-Di-[amino]-5-[2′,3′-di-(methoxy)-benzyl]-pyrimidin,

2,4-Di-[amino]-5-[3′,4′-(methylendioxy)-benzyl]-pyrimidin,

2,4-Di-[amino]-5-[3′-(methoxy)-4′-(äthoxy)-benzyl]-pyrimidin,

2,4-Di-[amino]-5-[3′-(methoxy)-4′-(allyloxy)-benzyl]-pyrimidin,

2,4-Di-[amino]-5-[3′-(methoxy)-4′-(n-butoxy)-benzyl]-pyrimidin,

2,4-Di-[amino]-5-[3′-(methoxy)-4′-(2″-{methoxy}-äthoxy)-benzyl]-pyrimidin,

2,4-Di-[amino]-5-[3′-(methoxy)-4′-(benzyloxy)-benzyl]-pyrimidin und/oder
2,4-Di-[amino]-5-[3′-(methoxy)-4′-(hydroxy)-benzyl]-pyrimidin
und/oder deren Säureadditionssalze sind.

9.) Verwendung nach Anspruch 1 oder 3 bis 8 oder 2,4-Di-(amino)-pyrimidinderivate nach Anspruch 2 bis 8, dadurch gekennzeichnet, daß die 2,4-Di-(amino)-pyrimidinderivate in Zubereitungsformen zur oralen, rektalen oder parenteralen Verabreichung vorliegen.

10.) Verwendung nach Anspruch 1 oder 3 bis 9 oder 2,4-Di-(amino)-pyrimidinderivate nach Anspruch 2 bis 9, dadurch gekennzeichnet, daß die 2,4-Di-(amino)-pyrimidinderivate in Form von Tabletten, Kapseln, Dragees, Lösungen, Suspensionen, Suppositorien oder Injektionspräparaten vorliegen.

Patentansprüche für die folgenden Vertragsstaaten: GR, ES

1. Verfahren zur Herstellung von antipyretisch, entzündungshemmend einschließlich antirheumatisch, anti-anginös und/oder antioxydierend wirkenden Arzneimitteln durch Vermischen von 5-(substituierten Benzyl) 2,4-Di-(amino)-pyrimidinderivaten der allgemeinen Formel

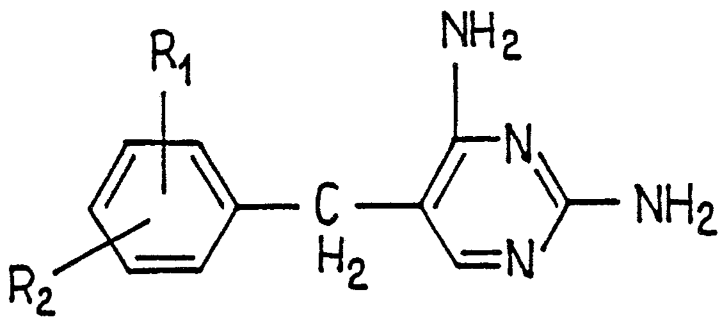

I ,

worin

$R_1$ und $R_2$ , die gleich oder verschieden sein können, für Wasserstoffatome, Hydroxygruppen, Alkoxyreste mit 1 bis 6 Kohlenstoffatom(en), Alkoxyalkoxyreste mit 1 bis 6 Kohlenstoffatom(en) in jedem Alkoxyteil, Alkenyloxyreste mit 2 bis 6 Kohlenstoffatomen beziehungsweise Phenylalkoxyreste mit 1 bis 3 Kohlenstoffatom(en) im Alkoxyteil stehen oder

$R_1$ und $R_2$ zusammen einen Alkylendioxyrest mit 1 oder 2 Kohlenstoffatom(en) darstellen,

mit der weiteren Maßgabe, daß

mindestens 1 von $R_1$ und $R_2$ von Wasserstoff verschieden ist,

beziehungsweise ihren Säureadditionssalzen mit inerten festen und/oder flüssigen Pharmazeutischen Trägern und/oder Hilfsstoffen.

2.) Verfahren zur Herstellung von 5-(substituierten Benzyl) 2,4-Di-(amino)-pyrimidinderivaten der allgemeinen Formel

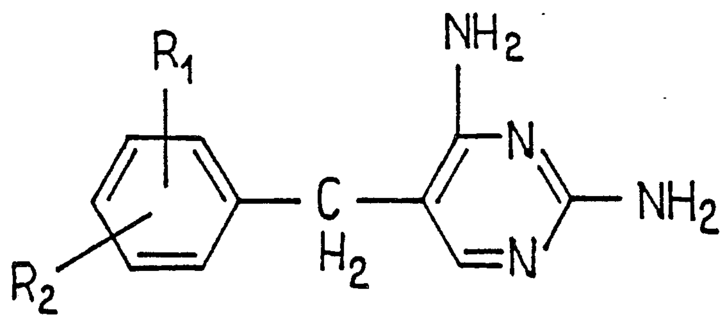

I ,

worin

$R_1$ und $R_2$ , die gleich oder verschieden sein können, für Wasserstoffatome, Hydroxygruppen, Alkoxyreste mit 1 bis 6 Kohlenstoffatom(en), Alkoxyalkoxyreste mit 1 bis 6 Kohlenstoffatom(en) in jedem Alkoxyteil, Alkenyloxyreste mit 2 bis 6 Kohlenstoffatomen beziehungsweise Phenylalkoxyreste mit 1 bis 3 Kohlenstoffatom(en) im Alkoxyteil stehen oder

$R_1$ und $R_2$ zusammen einen Äthylendioxyrest darstellen,

mit der weiteren Maßgabe, daß, im Falle daß das eine von $R_1$ und $R_2$ für Wasserstoff oder einen Alkoxy-oder Benzyloxyrest steht, das andere von $R_1$ und $R_2$ von Wasserstoff oder einem Alkoxy- oder Benzyloxyrest verschieden ist, sowie ihren Säureadditionssalzen, insbesondere zur Verwendung als Arzneimittel, dadurch gekennzeichnet, daß man in an sich bekannter Weise

a) substituierte $\beta$-(Cyan)-$\beta$-(methoxymethyl)-styrole der allgemeinen Formel

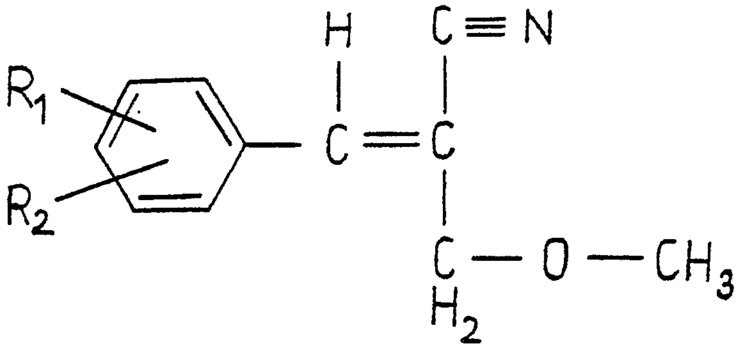

II ,

worin $R_1$ und $R_2$ die obigen Bedeutungen haben, mit Diäthylenglykol-mono-(niederalkyl)-äthern der allgemeinen Formel

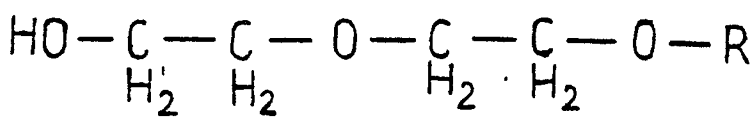

III ,

worin R für einen Alkylrest mit 1 bis 5, vorzugsweise 1 bis 4, Kohlenstoffatom(en) steht, umsetzt und die so erhaltenen substituierten 1-(Phenyl)-2-(cyan)-3-($\omega$-{alkoxy}-di-[äthoxy])-prop-3-ene der allgemeinen Formel

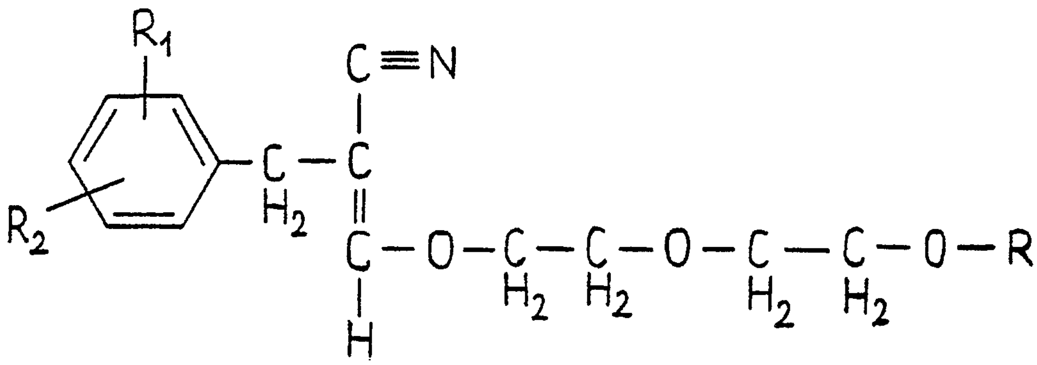

IV ,

worin R, $R_1$ und $R_2$ die obigen Bedeutungen haben, mit Guanidin oder dessen Säureadditionssalzen umsetzt oder

b) substituierte $\beta$-(Cyan)-$\beta$-(methoxymethyl)-styrole der allgemeinen Formel II mit Äthylenglykol-mono-(niederalkyl)-äthern der allgemeinen Formel

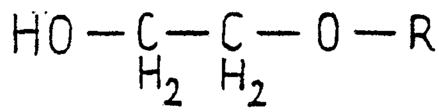

V ,

worin
R die obige Bedeutung hat,
umsetzt und die so erhaltenen, substituierten 1-(Phenyl)-2-(cyan)-3-(ω-{alkoxy}-äthoxy)-prop-3-ene der allgemeinen Formel

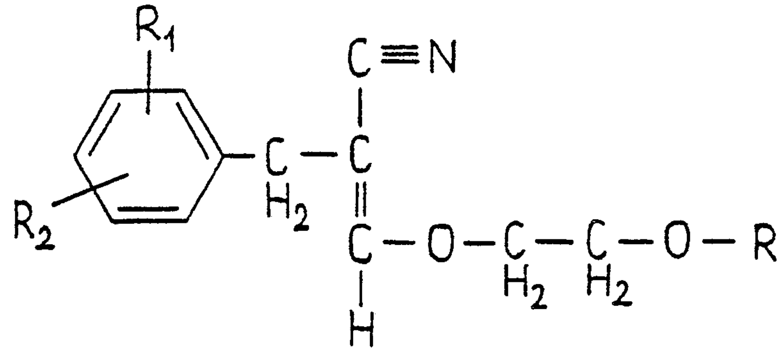

VI ,

worin
R, $R_1$ und $R_2$ die obigen Bedeutungen haben,
mit Guanidin oder dessen Säureadditionssalzen umsetzt
und gegebenenfalls in den erhaltenen 5-(substituierten Benzyl) 2,4-Di-(amino)-pyrimidinderivaten der allgemeinen Formel I [ein] Rest(e), für welche[n] $R_1$ und/oder $R_2$ stehen kann beziehungsweise können, in [einen] andere[n] umwandelt und/oder
gegebenenfalls die erhaltenen 5-(substituierten Benzyl) 2,4-Di-(amino)pyrimidinderivate der allgemeinen Formel I in Säureadditionssalze überführt und/oder die erhaltenen Säureadditionssalze der 5-(substituierten Benzyl) 2,4-Di-(amino)-pyrimidinderivate der allgemeinen Formel I in die freien 5-(substituierten Benzyl) 2,4-Di-(amino)pyrimidinderivatbasen und/oder andere Säureadditionssalze überführt.

3.) Verfahren nach Anspruch 1 oder 2, dadurch gekennzeichnet, daß man als 2,4-Di-(amino)-pyrimidinderivate solche, bei welchen der beziehungsweise die Alkoxyrest(e), für welche[n] $R_1$ und/oder $R_2$ stehen kann beziehungsweise können, [ein] solche[r] mit 1 bis 4, insbesondere 1 oder 2, Kohlenstoffatom(en) ist beziehungsweise sind, verwendet beziehungsweise herstellt.

4.) Verfahren nach Anspruch 1 bis 3, dadurch gekennzeichnet, daß man als 2,4-Di-(amino)-pyrimidinderivate solche, bei welchen der beziehungsweise die Alkoxyalkoxyrest(e), für welche[n] $R_1$ und/oder $R_2$ stehen kann beziehungsweise können, [ein] solche[r] mit 1 bis 4, insbesondere 1 und/oder 2, Kohlenstoffatom(en) in jedem Alkoxyteil ist beziehungsweise sind, verwendet beziehungsweise herstellt.

5.) Verfahren nach Anspruch 1 bis 4, dadurch gekennzeichnet, daß man als 2,4-Di-(amino)-pyrimidinderivate solche, bei welchen der beziehungsweise die Alkenyloxyrest(e), für welche[n] $R_1$ und/oder $R_2$ stehen kann beziehungsweise können, [ein] solche[r] mit 2 bis 4, insbesondere 2 oder 3, Kohlenstoffatomen ist beziehungsweise sind, verwendet beziehungsweise herstellt.

6.) Verfahren nach Anspruch 1 bis 5, dadurch gekennzeichnet, daß man als 2,4-Di-(amino)-pyrimidinderivate solche, bei welchen der beziehungsweise die Phenylalkoxyrest(e), für welche[n] $R_1$ und/oder $R_2$ stehen kann beziehungsweise können, [ein] solche[r] mit 1 oder 2 Kohlenstoffatom(en) im Alkoxyteil sind, verwendet beziehungsweise herstellt.

7.) Verfahren nach Anspruch 1 bis 6, dadurch gekennzeichnet, daß man als Säureadditionssalze 2,4-Di-(amino)-pyrimidinderivate der allgemeinen Formel I Hydrochloride verwendet beziehungsweise herstellt.

8.) Verfahren nach Anspruch 1 bis 7, dadurch gekennzeichnet, daß man als 2,4-Di-(amino)-pyrimidinderivate
2,4-Di-[amino]-5-[4′-(methoxy)-benzyl]-pyrimidin,
2,4-Di-[amino]-5-[3′-(methoxy)-benzyl]-pyrimidin,
2,4-Di-[amino]-5-[3′,5′-di-(methoxy)benzyl]-pyrimidin,
2,4-Di-[amino]-5-[2′,4′-di-(methoxy)-benzyl]-pyrimidin,

2,4-Di-[amino]-5-[2′,3′-di-(methoxy)-benzyl]-pyrimidin,
2,4-Di-[amino]-5-[3′,4′-(methylendioxy)-benzyl]-pyrimidin,
2,4-Di-[amino]-5-[3′-(methoxy)-4′-(äthoxy)-benzyl]-pyrimidin,
2,4-Di-[amino]-5-[3′-(methoxy)-4′-(allyloxy)-benzyl]-pyrimidin,
2,4-Di-[amino]-5-[3′-(methoxy)-4′-(n-butoxy)-benzyl]-pyrimidin,
2,4-Di-[amino]-5-[3′-(methoxy)-4′-(2″-{methoxy}-äthoxy)-benzyl]-pyrimidin,
2,4-Di-[amino]-5-[3′-(methoxy)-4′-(benzyloxy)-benzyl]-pyrimidin und/oder
2,4-Di-[amino]-5-[3′-(methoxy)-4′-(hydroxy)-benzyl]-pyrimidin
und/oder deren Säureadditionssalze verwendet beziehungsweise herstellt.